# FASCICULE DE BREVET EUROPEEN

(11) **EP 4 221 799 B1**
(45) Date de publication et mention de la délivrance du brevet: **07.01.2026**
(21) Numéro de dépôt: 21782999.3
(22) Date de dépôt: 29.09.2021
(51) Int. Cl.: A61M 25/02, A61B 17/02, A61M 25/06

(54) **DISPOSITIF DE FIXATION D'UN INTRODUCTEUR DE DISPOSITIF MÉDICAL DANS LE CORPS HUMAIN**
VORRICHTUNG ZUM BEFESTIGEN EINER EINFÜHRVORRICHTUNG EINES MEDIZINPRODUKTS IM MENSCHLICHEN KÖRPER
DEVICE FOR ATTACHING AN INTRODUCER OF A MEDICAL DEVICE IN THE HUMAN BODY

(30) Priorité: 29.09.2020 EP 20306113
(43) Date de publication de la demande: 09.08.2023
(73) Titulaire: Université de Strasbourg, 67000 Strasbourg (FR); Hôpitaux Universitaires de Strasbourg (HUS), 67000 Strasbourg (FR)
(72) Inventeur: CHAKFE, Nabil, 67150 Hindisheim (FR); NEUMANN, Nicole, 67400 Illkirch Graffenstaden (FR)
(74) Mandataire: Lavoix
(86) Numéro de dépôt international: PCT/EP2021/076768
(87) Numéro de publication internationale: WO 2022/069529

(56) Documents cités:
- EP-A1- 2 626 101
- EP-A1- 2 889 006
- WO-A1-01/68180
- WO-A1-2005/105194
- WO-A1-2013/109835
- WO-A1-2020/078601
- WO-A2-2004/037065
- WO-A2-2007/028007
- WO-A2-2007/117655
- US-A- 6 113 536
- US-A1- 2006 025 723
- US-A1- 2009 143 742
- US-A1- 2009 247 859

## Description

La présente invention concerne un dispositif de fixation d'un introducteur de dispositif médical dans le corps humain, comprenant :
- un support destiné à être maintenu sur le patient ou par rapport au patient ;
- un ensemble de liaison monté sur le support ;
- un étrier de réception de l'introducteur fixé à l'ensemble de liaison pour être monté sur le support, l'étrier définissant un passage central s'étendant suivant un axe longitudinal et une ouverture longitudinale d'insertion de l'introducteur dans le passage central ; et
- un système de maintien en position de l'introducteur dans l'étrier.

Le dispositif médical à introduire dans le corps humain est par exemple un implant formé par une endoprothèse ou une endovalve, et un outil de largage de l'implant.

Les endoprothèses sont utilisées par exemple pour réparer et supporter la paroi du conduit lorsque celui-ci est partiellement obstrué, ou pour ponter un anévrisme. Les endovalves sont utilisées pour remplacer une valve native malade ou défectueuse.

A cet effet, le dispositif de traitement portant l'implant est de préférence introduit par voie endoluminale, afin d'éviter d'ouvrir chirurgicalement le thorax du patient et de minimiser ainsi les risques opératoires, notamment pour des patients âgés ou faibles.

Dans le cas où le dispositif de traitement doit être conduit jusqu'à son lieu d'implantation à travers un vaisseau sanguin tel que l'aorte, il est connu de l'introduire dans le réseau sanguin à travers un vaisseau d'introduction tel que l'artère fémorale, en procédant à une incision au niveau de l'aine du patient.

D'autres vaisseaux d'introduction tels que l'artère carotide, les artères et les veines du membre supérieur, ou la veine jugulaire, sont aussi utilisés en fonction du lieu d'implantation final.

Un introducteur est alors nécessaire pour guider le dispositif de traitement depuis l'extérieur du corps à travers le vaisseau d'introduction jusqu'à un autre vaisseau sanguin de plus grand diamètre. Selon le dispositif de traitement à introduire, l'introducteur peut avoir un diamètre variant par exemple entre 4 F et 26 F (soit entre 1,3 mm et 8,7 mm).

L'introducteur possède une vanne d'étanchéité qui empêche le reflux sanguin hors du patient sous l'effet de la pression en réalisant une étanchéité autour du dispositif de traitement.

Lors de la procédure d'introduction du dispositif de traitement par le biais de l'introducteur, il subsiste toujours un risque de mobilisation de l'introducteur en dehors du vaisseau sanguin d'introduction. Ce risque peut survenir lors du retrait d'un dispositif de l'intérieur de l'introducteur comme une endoprothèse ou une sonde par exemple, ou lorsque la pression du flux de sang pulsé dans l'introducteur est trop importante.

Cette mobilisation de l'introducteur en dehors du vaisseau peut aller jusqu'à l'expulsion complète de l'introducteur et provoquer un saignement du patient par le point de ponction.

De plus, la mobilisation de l'introducteur en dehors du vaisseau peut entraîner le recul de l'introducteur, ce qui conduit à un mauvais positionnement du dispositif médical dans le patient.

Afin de minimiser les risques pour le patient, il est donc nécessaire de fixer l'introducteur pour éviter la mobilisation de celui-ci.

Habituellement, il est connu d'utiliser un opérateur afin de tenir manuellement l'introducteur fixe dans une position souhaitée tout au long de la procédure chirurgicale. Cependant, la procédure chirurgicale peut durer plusieurs heures et il existe donc un risque que l'opérateur bouge en raison de la fatigue. De plus, pendant toute la durée de la procédure, des images radiographiques sont prises pour vérifier le positionnement du dispositif médical dans le patient. L'opérateur qui maintient l'introducteur reste à proximité de la source d'émission de radiation des appareils de radiographie, ce qui est néfaste pour l'opérateur.

Il est également connu de fixer l'introducteur au patient à l'aide de points de suture. Cependant, cette solution ne permet pas d'adapter la position de l'introducteur au cours de la procédure. De plus, cette solution oblige à réaliser des points de suture supplémentaires sur le patient.

Afin d'éviter la mobilisation de l'introducteur, il est également connu par exemple de WO 2005 081882 A2 de suturer l'introducteur à un patch collé sur la peau du patient. Cependant, cette solution ne permet pas d'adapter la position de l'introducteur au cours de la procédure sans devoir changer de patch et mobiliser tout l'introducteur, ce qui engendre des risques supplémentaires pour le patient. Le document WO 01/68180 A1 divulgue un dispositif de fixation d'un cathéter.

Un but de l'invention est de fournir un système de fixation d'introducteur capable de maintenir de manière fiable, l'introducteur dans une position fixe souhaitée et permettant l'adaptation de cette position au cours de la procédure chirurgicale, sans engendrer de risque supplémentaire pour le patient.

Ces objectifs sont atteints au moyen d'un dispositif de fixation d'un introducteur de dispositif médical selon la revendication 1, ainsi qu'au moyen d'un procédé de fixation d'un dispositif de fixation de dispositif médical selon la revendication 16. Les revendication dépendantes 2-15 définissent les alternatives préférées.

Le dispositif de fixation divulgué ci-dessous peut comprendre l'une ou plusieurs caractéristiques suivantes, prise(s) isolement ou suivant toute combinaison techniquement possible :
- Le coussin de blocage comporte un manchon fendu le long d'une génératrice.
- Le coussin de blocage est formé d'un bloc en matériau déformable, ou d'une poche contenant un matériau déformable.
- Le système de maintien comprend un système de gonflage du coussin contrôlant le gonflage et le dégonflage du coussin.
- L'ensemble de liaison entre l'étrier et le support comporte une rotule et un support de rotule recevant la rotule.
- Le support comporte une barre, l'étrier de réception étant monté sur la barre du support par l'ensemble de liaison.
- L'étrier de réception est monté coulissant le long de la barre du support.
- La barre est extensible et la longueur de la barre est réglable.
- Le support comporte au moins une pièce supplémentaire fixée sur la barre afin d'augmenter la hauteur du support.
- Le support comporte un écarteur.
- Le support comporte un patch.
- L'étrier de réception comporte une chemise déformable entre une position ouverte et une position fermée.
- L'étrier de réception comporte un mécanisme de serrage ou/et de desserrage de la chemise déformable.
- Le mécanisme de serrage de la chemise déformable comporte un système à crans, un système de grenouillère ou un système de pinces.
- Il comprend un système de fixation de guide déporté par rapport à l'étrier de réception et au support, et avantageusement un lien souple raccordant le système de fixation de guide au support ou à l'étrier de réception.
- Le système de fixation de guide comprend un bloc de maintien fixé sur un patch définissant une fente de réception du guide.

Le procédé de fixation d'un introducteur de dispositif médical hors du corps humain divulgué ci-dessous comporte les étapes suivantes :
- fourniture d'un dispositif de fixation comportant un support préalablement fixé sur le patient et un étrier de réception de l'introducteur monté sur le support, l'étrier définissant un passage central s'étendant suivant un axe longitudinal et une ouverture longitudinale d'insertion de l'introducteur dans le passage central, le dispositif de fixation comportant un système de maintien en position de l'introducteur dans l'étrier, le système de maintien comportant au moins un coussin de blocage réversible de l'introducteur;
- mise en place de l'introducteur dans le passage central à travers l'ouverture longitudinale, et
- passage réversible du coussin de blocage d'une configuration relâchée lors de la mise en place de l'introducteur dans le passage central à une configuration de blocage en position de l'introducteur dans le passage central.

L'invention sera mieux comprise à la lecture de la description qui va suivre, donnée uniquement à titre d'exemple, et faite en se référant aux dessins annexés, sur lesquels :
- [Fig 1] la figure 1 est une vue en perspective d'un premier dispositif de fixation d'un introducteur de dispositif médical dans le corps humain.
- [Fig 2] la figure 2 est une vue en perspective de l'étrier de réception, de l'ensemble de liaison et du système de maintien du dispositif de fixation de la figure 1.
- [Fig 3] la figure 3 est une vue en perspective du support et de l'ensemble de liaison du dispositif de fixation de la figure 1.
- [Fig 4] la figure 4 est une vue en perspective d'un deuxième dispositif de fixation selon l'invention.
- [Fig 5] la figure 5 est une vue en perspective de la barre du support, de l'étrier de réception, de l'ensemble de liaison et du système de maintien du dispositif de fixation de la figure 4.
- [Fig 6] la figure 6 est une vue en perspective de l'étrier de réception, de l'ensemble de liaison et d'un système de maintien d'un troisième dispositif de fixation selon l'invention.
- [Fig 7] la figure 7 est une vue en perspective de l'étrier de réception, de l'ensemble de liaison et d'un système de maintien d'un quatrième dispositif de fixation selon l'invention.
- [Fig 8] la figure 8 est une vue en perspective de l'étrier de réception, de l'ensemble de liaison et d'un système de maintien d'un cinquième dispositif de fixation selon l'invention.
- [Fig 9] la figure 9 est une vue en perspective d'une variante de pièce intermédiaire comprise entre l'ensemble de liaison et le support du dispositif de fixation.
- [Fig 10] la figure 10 est une vue en perspective d'une variante du quatrième dispositif de fixation de la figure 7.
- [Fig 11] [Fig 12] [Fig 13] [Fig 14] [Fig 15] [Fig 16] les figures 11 à 16 sont des vues en perspective de dispositifs de fixation selon d'autres modes de réalisation de l'invention.

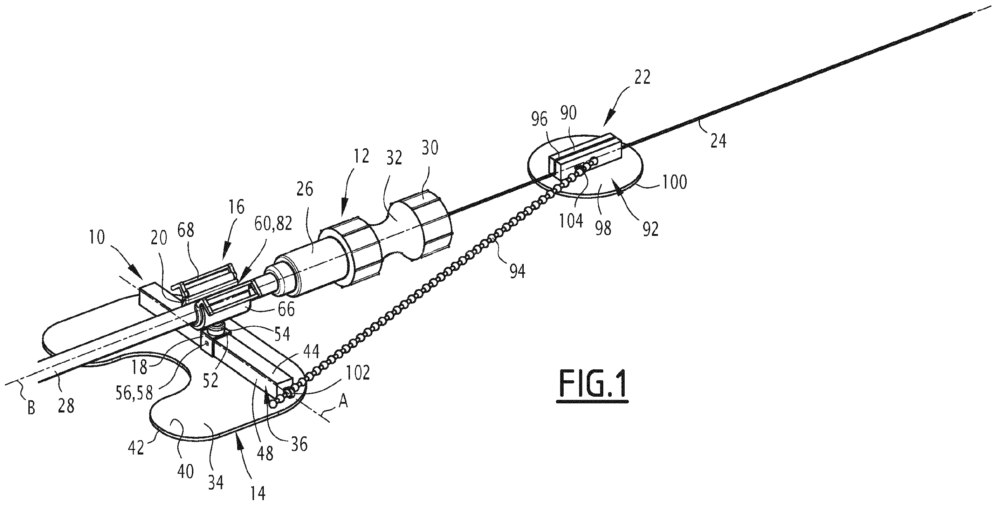

La figure 1 illustre un dispositif de fixation 10 d'un introducteur 12 de dispositif médical dans le corps humain.

Comme visible sur la figure 1, le dispositif de fixation 10 comprend un support 14, un étrier 16 de réception de l'introducteur 12, un ensemble de liaison 18 raccordant le support 14 à l'étrier 16 de réception et un système libérable de maintien 20 en position de l'introducteur 12 dans l'étrier 16. Le dispositif de fixation 10 comporte ici avantageusement un système de fixation 22 d'un guide chirurgical 24.

Par « libérable », on entend que le système de maintien 20 est propre à passer d'une configuration de blocage de l'introducteur 12, dans laquelle il retient fermement l'introducteur 12 en position, à une configuration relâchée dans laquelle il permet la mobilité de l'introducteur 12 au moins en translation suivant un axe.

L'introducteur 12 comprend un embout creux 26 d'introduction et un tube sensiblement rigide 28 de guidage du dispositif médical dans le vaisseau d'introduction.

L'embout 26 comprend un corps tubulaire 30 rigide de manipulation et une valve d'étanchéité 32 disposée dans le corps 30. La valve 32 est propre à être ouverte pour introduire le guide chirurgical 24 dans l'introducteur 12 puis le dispositif médical monté sur le guide chirurgical 24. Elle est propre à se refermer pour réaliser une étanchéité autour du guide 24.

Le guide chirurgical 24, destiné à naviguer dans le réseau sanguin, a ici été introduit selon la génératrice du tube 28 d'un point proximal de l'embout 26 se trouvant à l'extérieur du corps humain à un point distal de l'embout 26 se trouvant à l'intérieur du corps humain. Le guide chirurgical 24 est réalisé à base d'un fil métallique de faible diamètre, par exemple compris entre 0,1 mm et 3 mm.

Comme illustré sur la figure 1, le support 14 comprend un patch 34 de fixation et une barre 36 fixée transversalement sur le patch 34. Le support 14 est destiné à être fixé sur le patient à proximité de l'incision afin de définir un point fixe sur le corps du patient tout au long de la procédure chirurgicale.

Le patch 34 de fixation est un élément adhésif plat en forme de fer à cheval. Le patch 34 définit une première surface supérieure 40 non adhérente et une deuxième surface inférieure 42 adhésive propre à se coller sur la peau du patient.

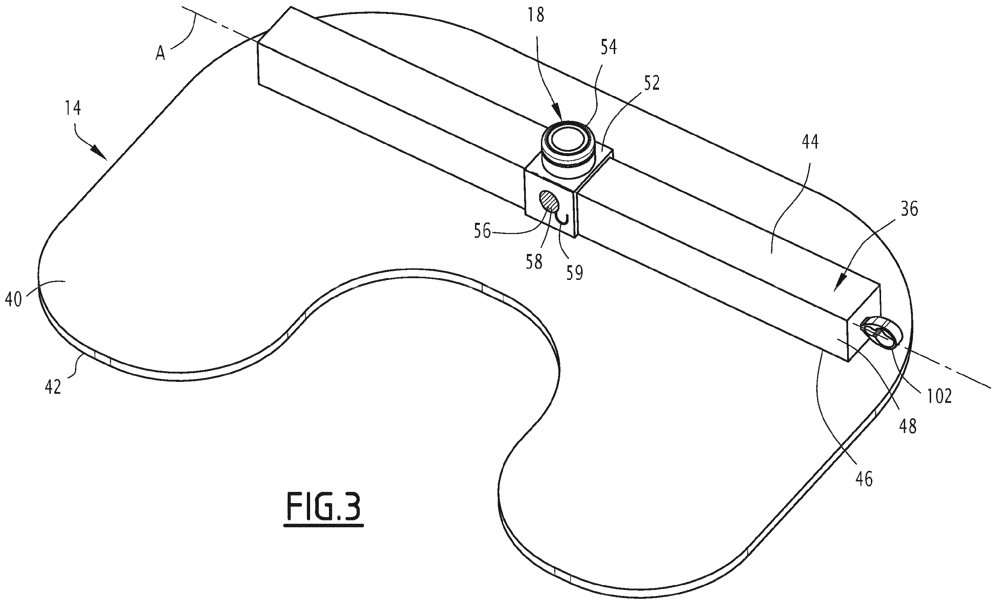

Comme cela est représenté sur la figure 3, la barre 36 est une pièce rectiligne de section rectangulaire s'étendant suivant un axe longitudinal A. La barre 36 définit une face supérieure 44, une face inférieure 46 et deux faces latérales 48. La face inférieure 46 de la barre 36 est fixée sur la surface supérieure 40 du patch 34.

La barre 36 est de préférence une barre métallique ou plastique à usage unique.

L'ensemble de liaison 18 comprend une attache 52 reliée à la barre 36 et un support de rotule 54 relié à l'étrier 16.

L'attache 52 est un cavalier en forme de U capable de se fixer sur la barre 36. L'attache 52 est montée sur la barre 36 et peut se déplacer en translation selon l'axe longitudinal A le long de la barre 36.

L'attache 52 comprend également un orifice taraudé 56 sur l'une de ses faces latérales capable de recevoir une vis de fixation 58 ou un autre système de fixation.

Le serrage de la vis de fixation 58 dans l'orifice 56 fixe la position de l'attache 52 le long de la barre 36.

La vis de fixation 58 est avantageusement liée à l'attache 52 par l'intermédiaire d'une liaison filiforme 59, par exemple en plastique, afin de ne pas être perdue en cas de détachement.

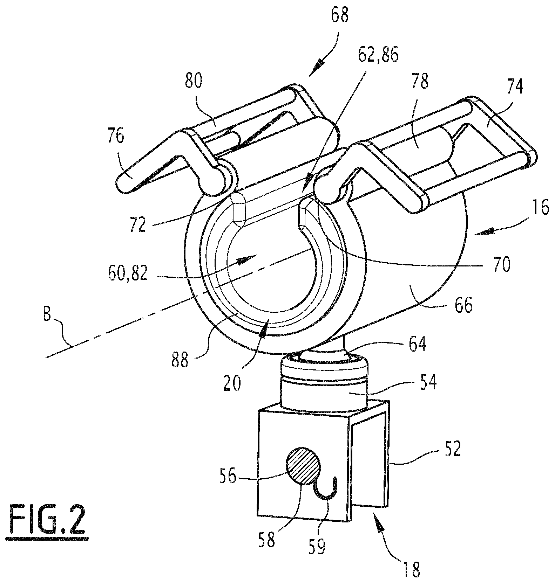

Comme cela est représenté sur la figure 2, l'étrier de réception 16 définit un passage central 60 s'étendant suivant un axe longitudinal B et une ouverture longitudinale 62 d'insertion de l'introducteur 12 dans le passage central 60, en forme de fente longitudinale qui débouche à ses extrémités longitudinales.

Sur la figure 1, l'axe longitudinal A de la barre 36 et l'axe longitudinal B du passage central 60 sont sensiblement perpendiculaires.

L'étrier de réception 16 comprend une rotule 64 reçue dans l'ensemble de liaison 18, une chemise déformable 66 et un mécanisme de desserrage 68 de la chemise déformable 66.

La rotule 64 se fixe dans le support de rotule 54 de l'ensemble de liaison 18, liant ainsi en liaison rotule l'étrier 16 et le support 14. Cette liaison rotule offre la possibilité d'orienter l'étrier 16, et donc l'introducteur 12, par rapport au support 14 fixé sur le corps du patient.

La chemise 66 s'étend autour du passage central 60 et définit un premier bord longitudinal 70 et un deuxième bord longitudinal 72 de part et d'autre de l'ouverture longitudinale 62.

La chemise 66 est déformable entre une position ouverte et une position fermée.

En position fermée, le premier bord 70 et le deuxième bord 72 de la chemise 66 sont au voisinage et/ou en contact l'un avec l'autre entraînant le rétrécissement de l'ouverture longitudinale 62 et donc l'impossibilité d'insérer l'introducteur 12 dans le passage central 60 ou de retirer l'introducteur 12 du passage central 60. Dans cet exemple, la position fermée est la position de repos.

En position ouverte, le premier bord 70 et le deuxième bord 72 de la chemise 66 sont à l'écart l'un de l'autre, entraînant l'ouverture de l'ouverture longitudinale 62 et donc rendant possible l'insertion et le déplacement de l'introducteur 12 dans le passage central 60 ou le retrait de l'introducteur 12 hors du passage central 60 à travers l'ouverture longitudinale 62.

Le mécanisme de desserrage 68 est manœuvrable par un praticien pour passer la chemise 66 de la position fermée à la position ouverte.

Dans cet exemple, le mécanisme de desserrage 68 comprend une première pince 74 montée rotative sur le premier bord 70 de la chemise 66 et une deuxième pince 76 montée rotative sur le deuxième bord 72 de la chemise 66.

Les deux pinces 74, 76 sont articulées en rotation par rapport à la chemise 66 autour d'axes parallèles à l'axe B. Lorsque les pinces 74, 76 s'appuient l'une sur l'autre, elles écartent le premier bord 72 du deuxième bord 74 et permettent d'ouvrir le système de maintien 20 pour libérer l'introducteur 12, comme décrit ci-dessous.

Le système de maintien 20 en position de l'introducteur 12 dans l'étrier 16 est monté fixement à l'intérieur de la chemise 66 de l'étrier de réception 16. Il est appliqué sur une surface intérieure de la chemise 66. Il s'étend à l'intérieur du passage central 60.

Le système de maintien 20 définit un passage central 82 s'étendant suivant l'axe longitudinal B du passage central 60 et de diamètre inférieur au passage central 60 de l'étrier 16. Le système de maintien 20 définit également une ouverture longitudinale 86 d'insertion de l'introducteur 12 dans le passage central 82 en continuité avec l'ouverture longitudinale 62 de l'étrier 16.

Dans cet exemple, le système de maintien 20 comprend un coussin de blocage 88 qui présente une forme de manchon cylindrique fendu suivant une génératrice.

Le coussin de blocage 88 est apte à recevoir l'introducteur 12 pour enserrer l'introducteur 12 dans le passage central 82 et le maintenir en position. Il est apte à se déformer pour libérer partiellement l'introducteur 12, notamment en translation le long de l'axe du passage central 82.

Le système de maintien 20 présente ainsi une configuration de blocage dans laquelle le coussin de blocage 88 est comprimé entre l'introducteur 12 et l'intérieur de la chemise 66 et une configuration relâchée, dans laquelle l'introducteur 12 est mobile au moins en translation dans le passage central 82.

Avantageusement, la texture surfacique du coussin de blocage 88 est rugueuse pour éviter le glissement de l'introducteur 12 dans le passage central 82 et pour lutter contre le traitement hydrophile des introducteurs qui est habituellement associé pour permettre la meilleure glisse dans les artères.

Le coussin de blocage 88 est par exemple réalisé d'un seul bloc à partir d'un matériau élastomère comme le silicone, le caoutchouc, le PVC ou est réalisé à l'aide d'une poche, par exemple en plastique, contenant un matériau déformable, par exemple de l'eau, de l'huile, de la gélatine ou du silicone liquide.

Comme cela est représenté sur la figure 1, le système de fixation 22 de guide 24 comprend un bloc de maintien 90 fixé sur un patch adhésif 92 et un lien souple 94 pour sa liaison au support 14.

Le bloc de maintien 90 définit une fente 96 de réception du guide 24. La fente 96 a un diamètre compris entre 0,1 mm et 3 mm selon le diamètre du guide 24 correspondant.

Avantageusement, le bloc de maintien 90 est réalisé en mousse.

Le patch adhésif 92 est un élément adhésif plat, ici de forme circulaire, définissant une surface supérieure 98 non adhérente et une surface inférieure 100 adhésive. Le bloc de maintien 90 est fixé sur la surface supérieure 98 du patch adhésif 92 par collage par exemple. La surface inférieure 100 du patch adhésif 92 est collée sur le patient.

Le système de fixation 22 est fixé sur le patient déporté par rapport au support 14, de sorte que la fente 96 de réception du guide 24 s'étende sensiblement selon l'axe longitudinal B de l'étrier de réception 16.

Le lien souple 94 est avantageusement un collier à bille fixé à une extrémité à un anneau 102 sur la barre 36 et à une autre extrémité à un anneau 104 sur le bloc de maintien 96.

Un procédé de fixation d'un introducteur 12 de dispositif médical dans le dispositif de fixation 10 décrit précédemment va maintenant être décrit.

En référence à la figure 1, le guide chirurgical 24 est tout d'abord inséré dans le patient jusqu'à sa position souhaitée.

Puis, l'introducteur 12 est mis en place en l'engageant sur le guide chirurgical 24. Le tube 28 est introduit partiellement dans le patient, l'embout 26 restant à l'extérieur du patient.

La partie du guide chirurgical 24 faisant saillie hors de l'introducteur 12 est ensuite introduite dans la fente 96 du bloc de maintien 90 définissant ainsi l'axe de centrage souhaité de l'introducteur 12.

Le support 14 sur lequel est monté l'étrier de réception 16 par le biais de l'ensemble de liaison 18 est ensuite fixé sur le patient en collant la surface inférieure 42 adhésive du patch 34 sur une surface de la peau du patient proche de l'incision d'insertion sans gêner l'espace de travail des praticiens.

L'introducteur 12 est alors inséré dans le passage central 82 à travers les ouvertures longitudinales 62, 86. Lors du passage dans les ouvertures 62, 86, l'introducteur 12 écarte le premier bord 70 du deuxième bord 72 passant la chemise 66 en position ouverte et le coussin 88 en configuration relâchée. Lorsque l'introducteur 12 atteint le passage 60, la chemise 66 repasse spontanément dans sa position fermée et le coussin 88 passe dans sa configuration de blocage dans laquelle il enserre l'introducteur 12.

L'étrier de réception 16 est ensuite translaté selon l'axe longitudinal A le long de la barre 36 et orienté par le biais de la rotule 64 lié à l'ensemble de liaison 18, de sorte que l'axe longitudinal B de l'étrier de réception 16 coïncide avec l'axe de centrage souhaité de l'introducteur 12.

La surface inférieure 100 adhésive du patch 92 est ensuite collée sur la peau du patient de telle manière que le lien souple 94 fixé d'un côté au support 14 et de l'autre au bloc de maintien 90 soit tendue et que la fente 96 de réception du guide 24 s'étende selon une direction parallèle à l'axe longitudinal B du passage central 60.

Dans cette configuration, le coussin de blocage 88 est en configuration de blocage. Le coussin de blocage 88 est comprimé entre l'introducteur 12 et l'intérieur de la chemise 66 et maintient ainsi l'introducteur 12 en position dans le passage central 60 de l'étrier 16.

L'introducteur 12 est maintenant fixe dans la position souhaitée. La mise en place du dispositif médical dans l'introducteur 12 est alors effectuée.

Lors de la procédure, il est possible si nécessaire d'ajuster la position de l'introducteur 12 dans le passage central 60 tout en restant centré par rapport au guide chirurgical 24.

Le praticien presse les deux pinces 74, 76 l'une contre l'autre. La chemise 66 est ainsi déformée pour écarter le premier bord 70 du deuxième bord 72 et passer dans la position ouverte. Le coussin de blocage 88 n'est donc plus comprimé entre l'introducteur 12 et l'intérieur de la chemise 66. Le coussin de blocage 88 est en configuration relâchée. L'introducteur 12 peut ainsi être mobilisé dans le passage central 60. En relâchant les deux pinces 74, 76, la chemise 66 retourne dans sa position fermée et le coussin de blocage 88 est de nouveau comprimé entre l'introducteur 12 et l'intérieur de la chemise 66 fixant ainsi l'introducteur 12 dans une nouvelle position.

Lorsque la mise en place est finie, l'introducteur 12 est retiré de l'étrier 16 et du corps du patient. Les patchs adhésifs 34, 92 sont ensuite décollés de la peau du patient.

Le dispositif de fixation 10 selon l'invention maintient donc l'introducteur 12 dans une position fixe souhaitée, tout en minimisant les risques pour le patient et pour le personnel médical.

De plus, la position de maintien de l'introducteur 12 dans le dispositif de fixation 10 est facilement adaptable au cours de la procédure chirurgicale, sans engendrer de risque supplémentaire pour le patient, tout en restant centré sur l'axe de centrage défini par le guide chirurgical 24.

Le dispositif 10 selon l'invention est en outre très versatile puisque le dispositif de fixation 10 est utilisable pour différents diamètres d'introducteur 12.

Grâce à l'utilisation de patchs adhésifs 34, 92, le procédé de fixation d'un introducteur 12 laisse uniquement des traces minimes et temporaires sur le corps du patient, à la différence des points de suture par exemple.

Dans une variante, le dispositif de fixation 10 comporte une pluralité de systèmes de fixation 22 du guide chirurgical 24, afin de fixer le guide 24 en plusieurs points.

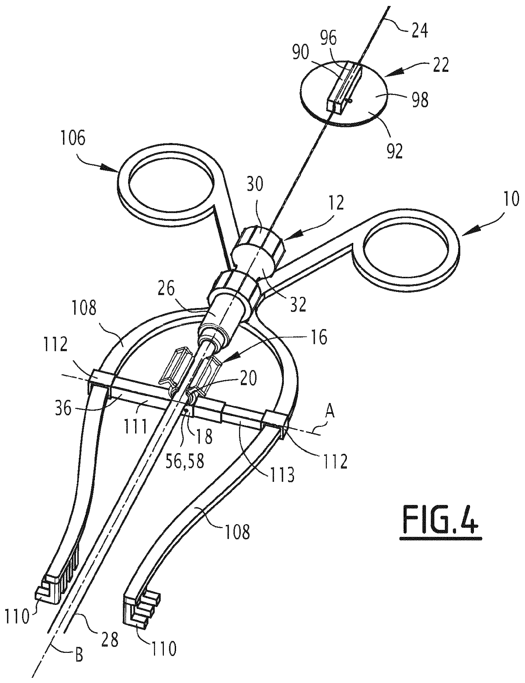

Dans une variante illustrée sur la figure 4, le support 14 comprend un écarteur 106. La barre 36 est fixée sur l'écarteur 106 au lieu d'être collée sur le patch 34.

L'écarteur 106 comprend deux branches 108 en regard l'une de l'autre et destinées à écarter chacune une des parois de l'incision d'insertion sur le corps du patient. Chaque branche 108 comporte une palette d'écartement 110 destinée à être positionnée en appui sur l'un des bords de l'incision.

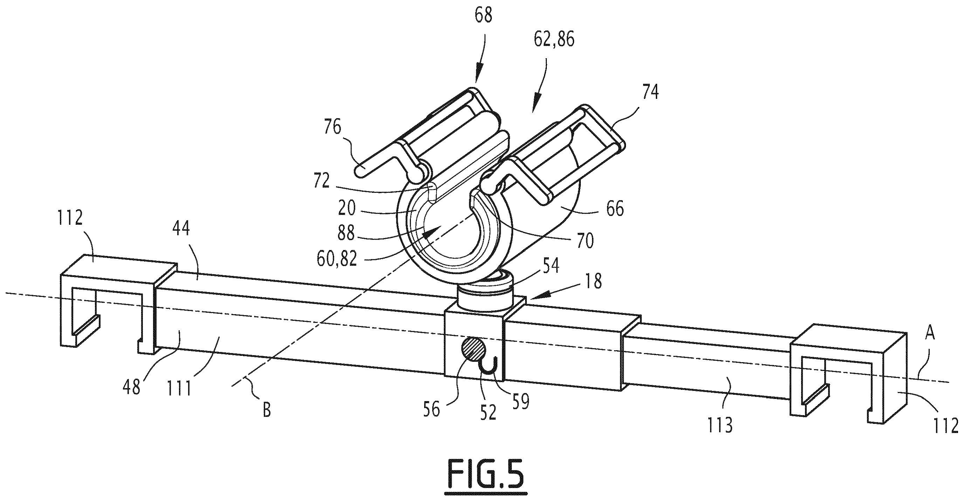

Comme cela est représenté sur la figure 5, la barre 36 comporte à chacune de ses extrémités un crochet 112 capable de se fixer sur les branches 108 de l'écarteur 106.

La barre 36 est extensible et la longueur de la barre 36 est réglable.

Avantageusement, la barre 36 comporte deux parties 111,113 montées de manière télescopique l'une dans l'autre. La longueur de la barre 36 est ainsi réglable, permettant ainsi d'adapter la taille de la barre 36 à l'écartement des branches 108 de l'écarteur 106.

Dans le mode de réalisation avec un écarteur 106, le procédé de fixation diffère de celui du mode de réalisation précédent uniquement pour l'étape de fixation du support 14 sur le corps du patient.

En effet, après avoir écarté les branches 108, chacune des palettes d'écartement 110 est fixée en appui sur l'un des bords de l'incision. L'écarteur 106 définit ainsi une position fixe sur le corps du patient.

La longueur de la barre 36 est ensuite réglée selon l'écartement des branches 108 de l'écarteur 106 en déplaçant les deux parties 111,113 l'une par rapport à l'autre. Les crochets 112 sont ensuite clipsés sur les branches 108 de l'écarteur 106. La barre 36 est ainsi fixée sur l'écarteur 106 définissant le support 14.

Avantageusement, la barre 36 est choisie métallique lors de la mise en place d'un écarteur fémoral chirurgical pour maintenir la voie précédemment ouverte.

Dans une variante, l'écarteur 106 choisi est un écarteur à usage unique avec une barre 36 déjà montée entre les deux branches 108 de l'écarteur 106. L'écarteur à usage unique définit donc en lui seul le support 14.

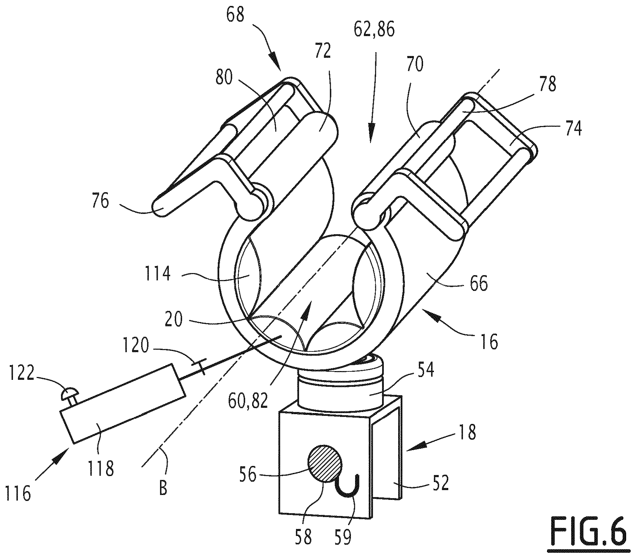

Selon un autre mode de réalisation illustré sur la figure 6, le coussin de blocage 88 du système de maintien 20 comprend au moins un ballonnet 114 capable de se gonfler par injection de fluide. Sur la figure 6, quatre ballonnets 114 sont représentés.

Avantageusement, la texture extérieure des ballonnets 114 est rugueuse pour éviter les glissements.

Le système de maintien 20 comprend un système de gonflage 116 des ballonnets 114 comprenant par exemple une seringue 118 propre à injecter du fluide dans les ballonnets 114 à travers une valve 120.

Avantageusement, le fluide injecté dans les ballonnets 114 est de l'eau, de l'huile, de la gélatine ou du silicone liquide.

Les ballonnets 114 ont un comportement de compression identique au coussin décrit précédemment.

Le système de gonflage 116 est contrôlé par un bouton pression 122. Une pression sur le bouton pression 122 permet d'ouvrir la valve 120 et d'injecter le liquide de la pompe 118 dans les ballonnets 114, entraînant le gonflage des ballonnets 114.

Dans une variante (non représentée), le mécanisme 68 est un mécanisme de serrage de la chemise 66. La chemise 66 n'est pas maintenue au repos dans la position fermée.

La première pince 74 présente une encoche, et la deuxième pince 76 comporte une butée propre à s'engager dans l'encoche.

Afin de passer la chemise 66 en position fermée, le praticien pivote la première pince 74 vers le haut et simultanément pivote la deuxième pince 76 vers le haut. Il met en contact les deux pinces 74, 76. La butée de la deuxième pince 76 s'insère ainsi dans l'encoche de la première pince 74, assurant le passage de la chemise 66 de la position ouverte à la position fermée.

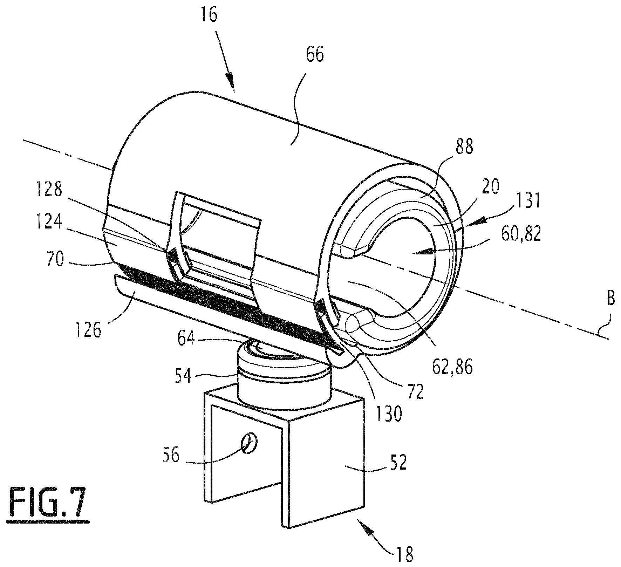

Dans un mode de réalisation représenté sur la figure 7, le mécanisme de serrage 68 de la chemise 66 ne comprend pas des pinces 74, 76 mais définit un système à crans comprenant une première partie 124 et une deuxième partie 126.

La première partie 124 du mécanisme de serrage 68 s'étend sur le premier bord 70 de la chemise 66 et définit une entaille 128 présentant intérieurement une denture interne.

La deuxième partie 126 du mécanisme de serrage 68 s'étend sur le deuxième bord 72 de la chemise 66 et définit une patte 130 présentant une denture externe. La patte 130 s'étend en regard de l'entaille 128 de la première partie 124 et destinée à s'insérer dans l'entaille 128.

L'insertion de la patte 130 dans l'entaille 128 par pression de la première partie 124 en direction de la deuxième partie 126 de le mécanisme de serrage 68 entraîne le contact du premier bord 70 avec le deuxième bord 72 de la chemise 66 et donc le passage de la chemise 66 en position fermée. La denture externe sur la patte 130 s'engage dans la denture interne de l'entaille 128 pour assurer un blocage en position.

La chemise 66 comprend une charnière 131 s'étendant selon une direction parallèle à l'axe longitudinal de la chemise 66. Avantageusement, la charnière 131 s'étend à l'opposé diamétralement de l'ouverture longitudinale 62.

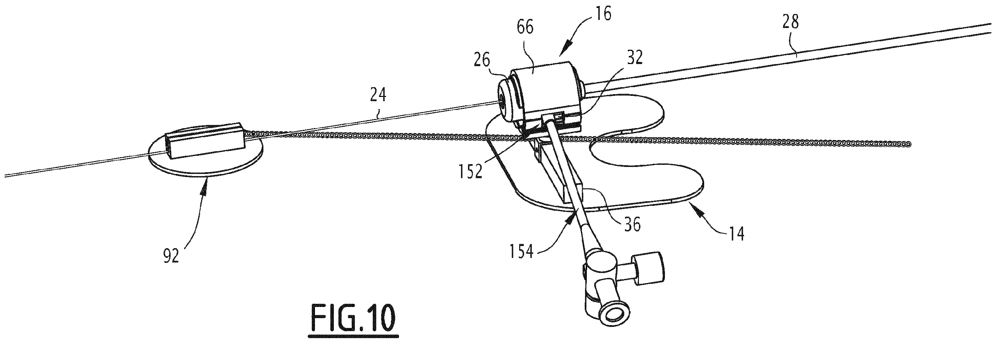

Dans une variante de ce mode de réalisation représentée sur la figure 10, l'étrier de réception 16 est apte à recevoir l'embout creux 26 de l'introducteur 12.

Avantageusement, la chemise 66 présente une encoche 152 propre à laisser passer un tuyau 154 d'introduction de fluide relié à la valve d'étanchéité 32 située à l'intérieur du corps tubulaire 30 de l'embout creux 26.

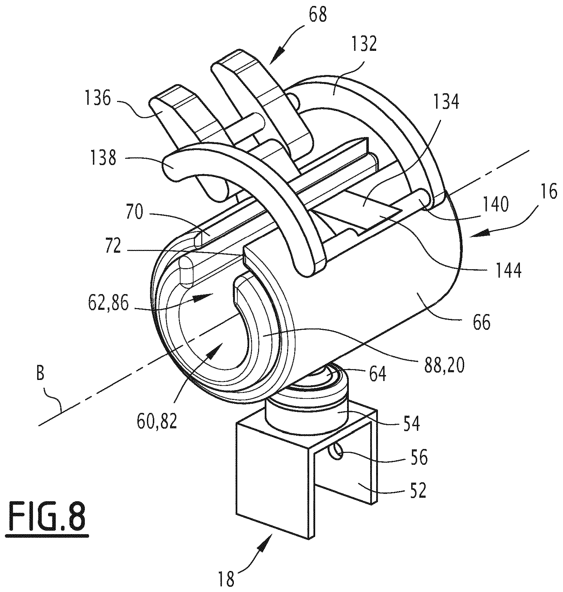

Selon un autre mode de réalisation illustré sur la figure 8, le mécanisme de serrage 68 de la chemise 66 définit un système à grenouillère comprenant une première partie 132 et une deuxième partie 134.

La première partie 132 définit une grenouillère et comprend un levier articulé 136 monté rotatif parallèlement à l'axe B sur le premier bord 70 de la chemise 66 et une biellette 138 articulée sur le levier 136 autour d'un axe parallèle à l'axe B. La biellette 138 comporte à son extrémité un lien de fermeture 140 sous forme d'une traverse s'étendant parallèlement à l'axe B.

La deuxième partie 134 est montée fixe sur le deuxième bord 72 de la chemise 66 et définit un crochet 144 capable de recevoir le lien de fermeture 140.

La première partie 132 définit une première position libre où elle s'étend relâchée sur le premier bord 70 de la chemise 66, une deuxième position engagée dans la deuxième partie, représentée sur la figure 8 où le lien de fermeture 140 est bloqué dans le crochet 144 et où le levier 136 est en position haute et une troisième position où le lien de fermeture 140 est bloqué dans le crochet 144 et où le levier 136 est en position basse.

Le passage de la deuxième position à la troisième position de la première partie 132 par pression sur le levier 136 contraint la biellette 138 à passer en position basse. La biellette 138 entraîne avec elle le deuxième bord 72 de la chemise 66 en direction du premier bord 70 et donc le passage de la chemise 66 en position fermée.

Le passage de la troisième position à la deuxième position de la première partie 132 en soulevant le levier 136 repasse la chemise 66 en position ouverte pour ajuster la position de l'introducteur 12 au cours de la procédure chirurgicale.

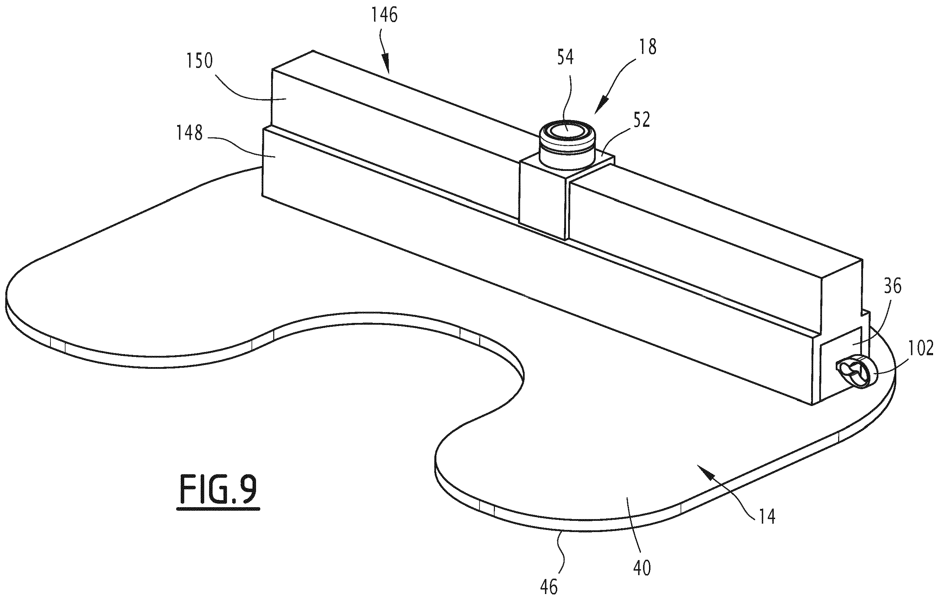

Dans une variante illustrée sur la figure 9, le support 14 comprend au moins une pièce supplémentaire 146 se fixant sur la barre 36 afin d'augmenter la hauteur du support 14.

La pièce supplémentaire 146 ou l'empilement de pièces 146 augmente la hauteur du support 14 et ainsi positionne l'introducteur 12 à une hauteur souhaitée afin, par exemple, de maintenir l'angle de l'introducteur à la sortie de l'incision.

La pièce 146 comprend une première partie 148 définissant une empreinte creuse destinée à recevoir la barre 36 et une deuxième partie 150 de dimension identique à la barre 36 et destinée à recevoir l'ensemble de liaison 18.

La pièce 146 est montée sur la barre 36 en clipsant la première partie 148 de la pièce 146 sur la barre 36. L'ensemble de liaison 18 est monté sur la deuxième partie 180 de la pièce 146.

Une première pièce 146 peut être empilée sur une deuxième pièce 146 en clipsant la première partie 148 de la première pièce 146 sur la deuxième partie 150 de la deuxième pièce 146. Cette opération peut être réalisée autant de fois que nécessaire afin d'obtenir un empilement de pièces 146 avec la hauteur souhaitée.

L'empilement de pièces 146 est fixé sur la barre 36 en clipsant la première partie 148 de la pièce 146 se trouvant en bas de la pile sur la barre 36 et l'ensemble de liaison 18 est monté sur la deuxième partie 150 de la pièce 146 se trouvant en haut de la pile.

En variante, un autre système de rehausseur est utilisé à la place de l'empilement de pièces 146, par exemple un système à vis.

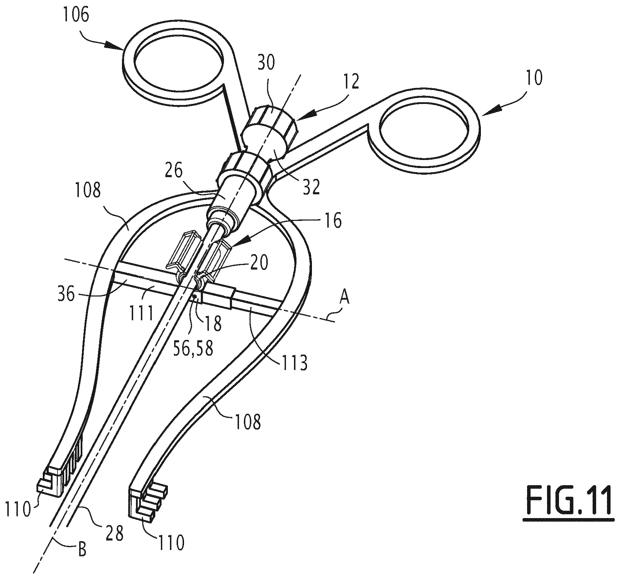

Dans la variante de l'invention illustrée sur la figure 11, le support 14 comporte un écarteur 106 à usage unique avec une barre 36 déjà montée entre les deux branches 108 de l'écarteur 106. Un tel écarteur 106 à usage unique est par exemple fabriqué en un matériau plastique.

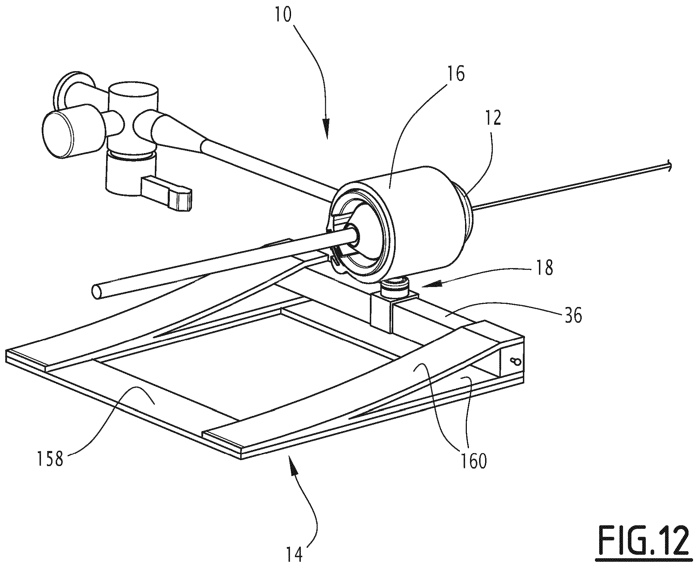

Dans le mode de réalisation de l'invention illustré sur la figure 12, le support 14 comporte un champ stérile constitué d'un film adhésif 158 définissant une barrière stérile entre sa surface inférieure collée sur la peau du patient et sa surface. Le film adhésif 158 présente une fenêtre centrale traversante.

Dans ce mode de réalisation, chaque extrémité de la barre 36 est montée entre deux pattes métalliques 160 de maintien qui sont fixées sur le film adhésif 158.

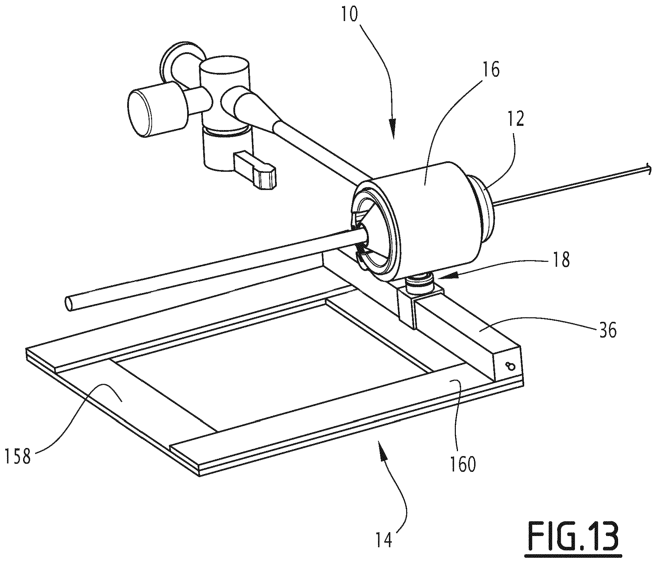

Dans la variante de ce mode de réalisation illustrée sur la figure 13, chaque extrémité de la barre 36 est montée sur une unique patte métallique 160 de maintien fixée sur le film adhésif 158.

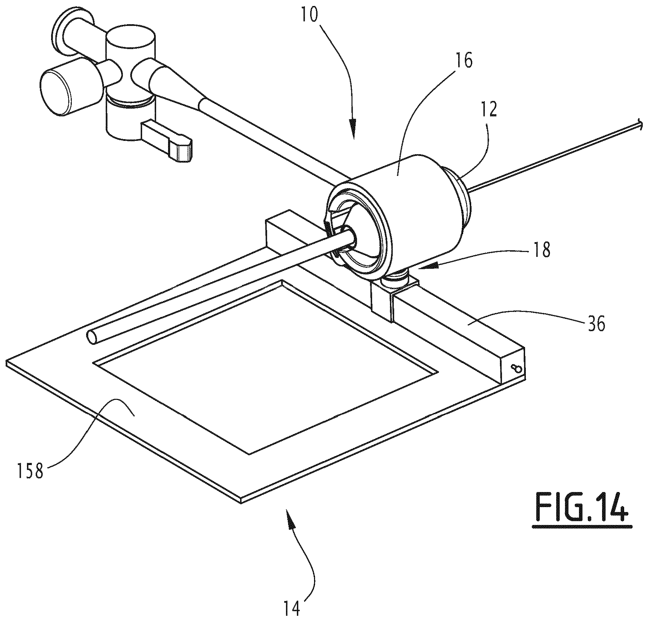

Dans la variante illustrée sur la figure 14, la barre 36 est directement collée sur le film adhésif du champ stérile.

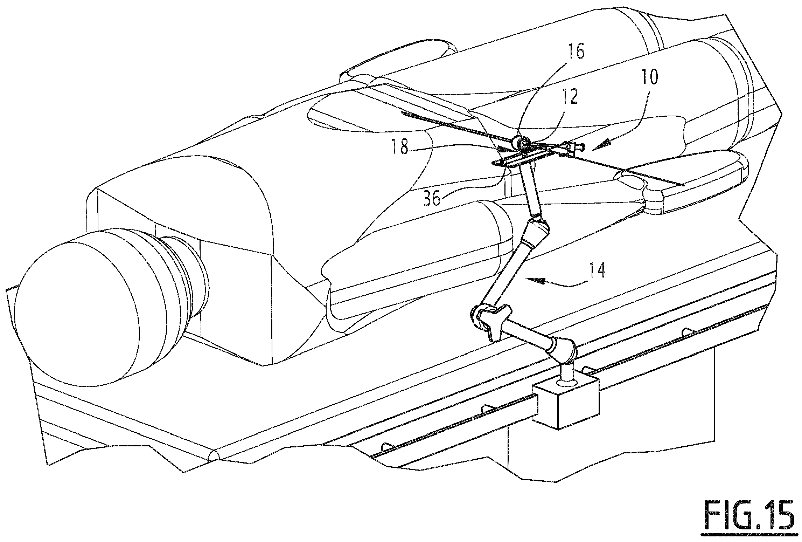

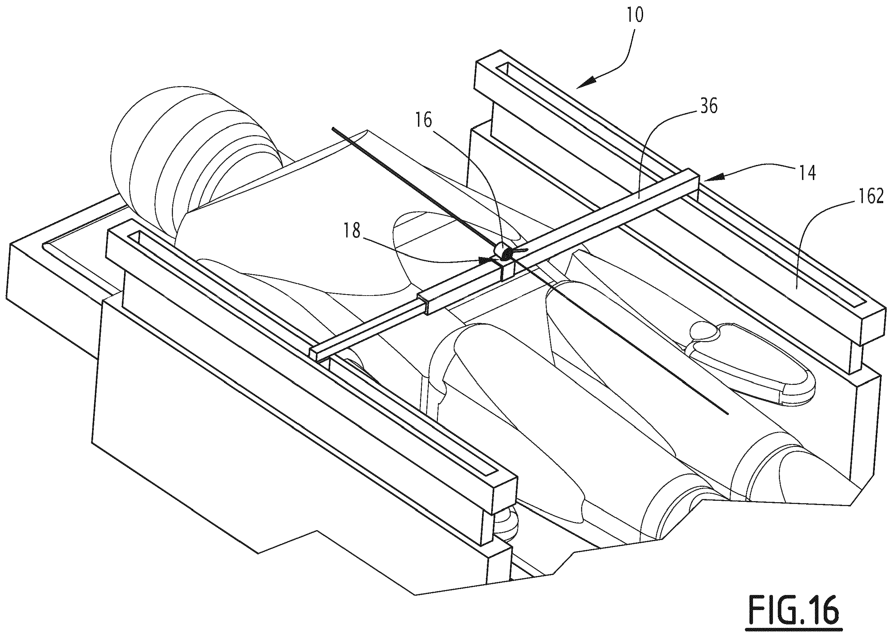

En variante de l'invention illustrée sur les figures 15 et 16, le support 14 n'est pas maintenu directement sur le patient, mais est maintenu de manière fixe par rapport au patient.

Par « maintenu de manière fixe par rapport au patient », on entend notamment que le patient est immobilisé sur une surface, par exemple d'une table d'opération, le support 16 étant maintenu fixe par rapport à la table et par suite, par rapport au patient.

Dans le mode de réalisation illustré sur la figure 15, le support 14 comporte un bras articulé fixé en l'une de ses extrémités à la table d'opération sur laquelle est allongé le patient. La barre 36 est fixée sur l'autre extrémité du bras articulé et est apte à être positionnée et maintenue par l'intermédiaire du bras articulé dans une position souhaitée par rapport au patient. Le bras articulé est de préférence radio-transparent.

Dans le mode de réalisation illustré sur la figure 16, le support 14 comporte une coque rigide comprenant deux parois latérales 162 mobiles. La coque rigide comprenant les parois latérales 162 est de préférence radio-transparente.

Le patient est reçu sur la coque rigide entre les deux parois latérales 162, qui sont déplacées vers le patient pour venir en butée contre le patient, de part et d'autre du patient. La barre 36 s'étend entre les deux parois latérales 162 et forme une ceinture au-dessus-du patient. La barre 36 est par exemple montée coulissante sur des rails définis sur chacune de parois latérales 162. De manière optionnelle, la barre 36 est également fixée en chacune de ses extrémités par un patch adhésif sur la peau du patient.

Dans une variante de l'invention, le dispositif de fixation 10 comprend une pluralité de systèmes de fixation 22 de guide. Chacun des systèmes de fixation 22 de guide est adapté à la fixation d'un type et/ou diamètre de guide chirurgical 24. Les systèmes de fixation 22 de guide diffèrent les uns des autres par leur couleur et/ou leur texture, de sorte qu'ils soient faciles à différencier et à identifier.

## Revendications

1. Dispositif de fixation (10) d'un introducteur (12) de dispositif médical dans le corps humain comprenant :
- un support (14) destiné à être maintenu sur le patient ou par rapport au patient ;
- un ensemble de liaison (18) monté sur le support (14) ;
- un étrier de réception (16) de l'introducteur (12) fixé à l'ensemble de liaison (18) pour être monté sur le support (14), l'étrier (16) définissant un passage central (60) s'étendant suivant un axe longitudinal (B) et une ouverture longitudinale (62) d'insertion de l'introducteur (12) dans le passage central (60) ;
- un système de maintien (20) en position de l'introducteur (12) dans l'étrier (16) ;
système de maintien (20) comportant au moins un coussin de blocage (88) réversible de l'introducteur (12), le système de maintien (20) étant propre à passer d'une configuration relâchée pour la mise en place, le déplacement et le retrait de l'introducteur (12) dans le passage central (60) à une configuration de blocage en position de l'introducteur (12) dans le passage central (60), le support (14) comportant une barre (36), l'étrier de réception (16) étant monté sur la barre (36) du support (14) par l'ensemble de liaison (18), l'étrier de réception (16) étant monté coulissant le long de la barre (36) du support (14).

2. Dispositif de fixation (10) selon la revendication 1, dans lequel le coussin de blocage (88) comporte un manchon fendu le long d'une génératrice.

3. Dispositif de fixation (10) selon la revendication 1 ou 2, dans lequel le coussin de blocage (88) est formé d'un bloc en matériau déformable, ou d'une poche contenant un matériau déformable.

4. Dispositif de fixation (10) selon la revendication 1 ou 2, dans lequel le système de maintien (20) comprend un système de gonflage (116) du coussin (88) contrôlant le gonflage et le dégonflage du coussin (88).

5. Dispositif de fixation (10) selon l'une quelconque des revendications précédentes, dans lequel l'ensemble de liaison (18) entre l'étrier (16) et le support (14) comporte une rotule (64) et un support de rotule (54) recevant la rotule (64).

6. Dispositif de fixation (10) selon l'une quelconque des revendications précédentes, dans lequel la barre (36) est extensible et la longueur de la barre (36) est réglable.

7. Dispositif de fixation (10) selon l'une quelconque des revendication précédentes, dans lequel le support (14) comporte au moins une pièce supplémentaire (146) fixée sur la barre (36) afin d'augmenter la hauteur du support (14).

8. Dispositif de fixation (10) selon l'une quelconque des revendications précédentes, dans lequel le support (14) comporte un écarteur (106).

9. Dispositif de fixation (10) selon l'une quelconque des revendications précédentes, dans lequel le support (14) comporte un patch (34).

10. Dispositif de fixation (10) selon l'une quelconque des revendications précédentes, dans lequel l'étrier de réception (16) comporte une chemise (66) déformable entre une position ouverte et une position fermée.

11. Dispositif de fixation (10) selon la revendication 10, dans lequel l'étrier de réception (16) comporte un mécanisme de serrage ou/et de desserrage (68) de la chemise déformable (66).

12. Dispositif de fixation (10) selon la revendication 11, dans lequel le mécanisme de serrage (68) de la chemise déformable (66) comporte un système à crans, un système de grenouillère ou un système de pinces.

13. Dispositif de fixation (10) selon l'une quelconque des revendications précédentes, comprenant un système de fixation (22) de guide déporté par rapport à l'étrier de réception (16) et au support (14), et avantageusement un lien souple (94) raccordant le système de fixation (22) de guide au support (14) ou à l'étrier de réception (16).

14. Dispositif de fixation (10) selon la revendication 13, dans lequel le système de fixation (22) de guide comprend un bloc de maintien (90) fixé sur un patch (92) définissant une fente (96) de réception du guide (24).

15. Dispositif de fixation (10) selon l'une quelconque des revendications précédentes, dans lequel le support (14) comporte un champ stérile, un bras articulé, ou une coque rigide.

16. Procédé de fixation d'un introducteur (12) de dispositif médical, mis en oeuvre hors du corps humain, comportant les étapes suivantes :
- fourniture d'un dispositif de fixation (10) comportant un support (14) préalablement fixé sur le patient ou par rapport au patient et un étrier de réception (16) de l'introducteur (12) monté sur le support (14), l'étrier (16) définissant un passage central (60) s'étendant suivant un axe longitudinal (B) et une ouverture longitudinale (62) d'insertion de l'introducteur (12) dans le passage central (60), le dispositif de fixation (10) comportant un système de maintien (20) en position de l'introducteur (12) dans l'étrier (16), le système de maintien (20) comportant au moins un coussin de blocage (88) réversible de l'introducteur (12), le support (14) comportant une barre (36), l'étrier de réception (16) étant monté sur la barre (36) du support (14) par l'ensemble de liaison (18), l'étrier de réception (16) étant monté coulissant le long de la barre (36) du support (14) ;
- mise en place de l'introducteur (12) dans le passage central (60) à travers l'ouverture longitudinale (62),
- passage réversible du coussin de blocage (88) d'une configuration relâchée lors de la mise en place de l'introducteur (12) dans le passage central (60) à une configuration de blocage en position de l'introducteur (12) dans le passage central (60).

## Patentansprüche

1. Befestigungsvorrichtung (10) einer Einführvorrichtung (12) eines Medizinprodukts in den menschlichen Körper, umfassend:
- eine Halterung (14), die dazu bestimmt ist, am Patienten oder in Bezug auf den Patienten gehalten zu werden;
- eine Verbindungsanordnung (18), die an der Halterung (14) montiert ist;
- einen Aufnahmebügel (16) für die Einführvorrichtung (12), die an der Verbindungsanordnung (18) befestigt ist, um an der Halterung (14) montiert zu werden, wobei der Bügel (16) einen mittleren Durchgang (60), der sich entlang einer Längsachse (B) erstreckt, und eine Längsöffnung (62) zum Einführen der Einführvorrichtung (12) in den mittleren Durchgang (60) definiert;
- ein Haltesystem (20) das die Einführvorrichtung (12) in dem Bügel (16) in Position hält;
das Haltesystem (20) umfassend
mindestens ein reversibles
Sperrkissen (88) für die Einführvorrichtung (12), wobei das Haltesystem (20) geeignet ist, um von einer entspannten Konfiguration zum Einsetzen, Verschieben und Zurückziehen der Einführvorrichtung (12) in den mittleren Durchgang (60) in eine Konfiguration zum Blockieren der Position der Einführvorrichtung (12) in dem mittleren Durchgang (60) überzugehen, die Halterung (14) umfassend eine Stange (36), wobei der Aufnahmebügel (16) durch die Verbindungsanordnung (18) an der Stange (36) der Halterung (14) montiert ist, wobei der Aufnahmebügel (16) verschiebbar entlang der Stange (36) der Halterung (14) montiert ist.

2. Befestigungsvorrichtung (10) nach Anspruch 1, wobei das Sperrkissen (88) eine Hülse aufweist, die entlang einer Mantellinie geschlitzt ist.

3. Befestigungsvorrichtung (10) nach Anspruch 1 oder 2, wobei das Sperrkissen (88) aus einem Block aus verformbarem Material oder aus einer Tasche, die ein verformbares Material enthält, gebildet ist.

4. Befestigungsvorrichtung (10) nach Anspruch 1 oder 2, wobei das Haltesystem (20) ein Aufblassystem (116) für das Kissen (88) umfasst, das ein Aufblasen und Entleeren des Kissens (88) steuert.

5. Befestigungsvorrichtung (10) nach einem der vorherigen Ansprüche, wobei die Verbindungsanordnung (18) zwischen dem Bügel (16) und der Halterung (14) einen Kugelkopf (64) und eine Kugelkopfhalterung (54), die den Kugelkopf (64) aufnimmt, umfasst.

6. Befestigungsvorrichtung (10) nach einem der vorherigen Ansprüche, wobei die Stange (36) ausziehbar ist und die Länge der Stange (36) einstellbar ist.

7. Befestigungsvorrichtung (10) nach einem der vorherigen Ansprüche, wobei die Halterung (14) mindestens ein zusätzliches Teil (146) aufweist, das an der Stange (36) befestigt ist, um die Höhe der Halterung (14) zu vergrößern.

8. Befestigungsvorrichtung (10) nach einem der vorherigen Ansprüche, wobei die Halterung (14) einen Abstandhalter (106) umfasst.

9. Befestigungsvorrichtung (10) nach einem der vorherigen Ansprüche, wobei die Halterung (14) ein Patch (34) umfasst.

10. Befestigungsvorrichtung (10) nach einem der vorherigen Ansprüche, wobei der Aufnahmebügel (16) einen Mantel (66) umfasst, der zwischen einer offenen und einer geschlossenen Position verformbar ist.

11. Befestigungsvorrichtung (10) nach Anspruch 10, wobei der Aufnahmebügel (16) einen Mechanismus zum Spannen oder/und Lösen (68) des verformbaren Mantels (66) umfasst.

12. Befestigungsvorrichtung (10) nach Anspruch 11, wobei der Spannmechanismus (68) des verformbaren Mantels (66) ein Rastsystem, ein Schnallensystem oder ein Klammersystem umfasst.

13. Befestigungsvorrichtung (10) nach einem der vorherigen Ansprüche, umfassend ein Befestigungssystem (22) zum Führen, das von dem Aufnahmebügel (16) und der Halterung (14) versetzt ist, und vorteilhafterweise eine flexible Verbindung (94), die das Befestigungssystem (22) zum Führen mit der Halterung (14) oder dem Aufnahmebügel (16) verbindet.

14. Befestigungsvorrichtung (10) nach Anspruch 13, wobei das Befestigungssystem (22) zum Führen einen Halteblock (90) umfasst, der an einem Patch (92) befestigt ist, das einen Schlitz (96) zum Aufnehmen der Führung (24) definiert.

15. Befestigungsvorrichtung (10) nach einem der vorherigen Ansprüche, wobei die Halterung (14) ein steriles Tuch, einen Gelenkarm oder eine starre Schale umfasst.

16. Verfahren zum Befestigen einer Einführvorrichtung (12) eines Medizinprodukts, die außerhalb des menschlichen Körpers eingesetzt wird, umfassend die folgenden Schritte:
- Bereitstellen einer Befestigungsvorrichtung (10), umfassend eine Halterung (14), die zuvor an dem Patienten oder in Bezug auf den Patienten befestigt wurde, und einen Bügel (16) zum Aufnehmen der Einführvorrichtung (12), die auf dem Halterung (14) montiert ist, wobei der Bügel (16) einen mittleren Durchgang (60), der sich entlang einer Längsachse (B) erstreckt, und eine Längsöffnung (62) zum Einführen der Einführvorrichtung (12) in den mittleren Durchgang (60) definiert, die Befestigungsvorrichtung (10) umfassend ein System (20) zum Halten der Einführvorrichtung (12) in Position in dem Bügel (16), wobei das Haltesystem (20) mindestens ein reversibles Sperrkissen (88) der Einführvorrichtung (12) umfasst, die Halterung (14) eine Stange (36) umfasst, der Aufnahmebügel (16) durch die Verbindungsanordnung (18) an der Stange (36) der Halterung (14) montiert ist, wobei der Aufnahmebügel (16) entlang der Stange (36) der Halterung (14) verschiebbar monteirt ist;
- Einsetzen der Einführvorrichtung (12) in den mittleren Durchgang (60) durch die Längsöffnung (62),
- reversibles Übergehens des Sperrkissens (88) von einer entspannten Konfiguration beim Einsetzen der Einführvorrichtung (12) in den mittleren Durchgang (60) zu einer Konfiguration zum Sperren der Position der Einführvorrichtung (12) in dem mittleren Durchgang (60).

## Claims

1. A device for anchoring (10) an introducer (12) of a medical device in the human body comprising:
- a support (14) intended to be held on the patient or relative to the patient ;
- a connecting assembly (18) mounted on the support (14); and
- a receiving clip (16) of the introducer (12) attached to the connecting assembly (18) for mounting on the support (14), the clip (16) defining a central passage (60) extending along a longitudinal axis (B) and a longitudinal opening (62) for insertion of the introducer (12) into the central passage (60);
- a holding system (20) for holding the introducer (12) in position in the clip (16);
the holding system (20) including at least one reversible locking pad (88) for the introducer (12), the holding system (20) being able to change from a released configuration for the positioning, the displacement and the removal of the introducer (12) in the central passage (60) to a configuration for locking the introducer (12) in position in the central passage (60), the support (14) including a bar (36), the receiving clip (16) being mounted on the bar (36) of the support (14) by the connecting assembly (18), the receiving clip (16) being slidably mounted along the bar (36) of the support (14).

2. The anchoring device (10) according to claim 1, wherein the locking pad (88) includes a sleeve split along a generatrix.

3. The anchoring device (10) according to claim 1 or 2, wherein the locking pad (88) is formed of a block of deformable material, or a bag containing deformable material.

4. The anchoring device (10) according to claim 1 or 2, wherein the holding system (20) comprises an inflation system (116) for the pad (88) controlling the inflation and deflation of the pad (88).

5. The anchoring device (10) according to any one of the preceding claims, wherein the connecting assembly (18) between the clip (16) and the support (14) includes a ball (64) and a ball joint (54) receiving the ball (64).

6. The anchoring device (10) according to any one of the preceding claims, wherein the bar (36) is extendable and the length of the bar (36) is adjustable.

7. The anchoring device (10) according to any one of the preceding claims, wherein the support (14) includes at least one additional piece (146) attached to the bar (36) to increase the height of the support (14).

8. The anchoring device (10) according to any one of the preceding claims, wherein the support (14) includes a spacer (106).

9. The anchoring device (10) according to any one of the preceding claims, wherein the support (14) includes a patch (34).

10. The anchoring device (10) according to any one of the preceding claims, wherein the receiving clip (16) includes a sleeve (66) deformable between an open position and a closed position.

11. The anchoring device (10) according to claim 10, wherein the receiving clip (16) includes a mechanism (68) for clamping and/or unclamping the deformable sleeve (66).

12. The anchoring device (10) according to claim 11, wherein the clamping mechanism (68) of the deformable sleeve (66) comprises a notch system, a toggle system or a clamp system.

13. The anchoring device (10) according to any one of the preceding claims, comprising a guide fixing system (22) remote from the receiving clip (16) and the support (14), and advantageously a flexible link (94) connecting the guide fixing system (22) to the support (14) or to the receiving clip (16).

14. The anchoring device (10) according to claim 13, wherein the guide fixing system (22) comprises a holding block (90) attached to a patch (92) defining a slot (96) for receiving the guide (24).

15. The anchoring device (10) according to any one of the preceding claims, wherein the support (14) includes a sterile field, an articulated arm, or a rigid shell.

16. A method for anchoring an introducer (12) of a medical device, implemented outside the human body, comprising the following steps:
- providing a anchoring device (10) including a support (14) previously fixed to the patient or relative to the patient and a clip (16) for receiving the introducer (12) mounted on the support (14), the clip (16) defining a central passage (60) extending along a longitudinal axis (B) and a longitudinal opening (62) for inserting the introducer (12) into the central passage (60), the anchoring device (10) including a holding system (20) for holding the introducer (12) in position in the clip (16), the holding system (20) including at least one reversible locking pad (88) for the introducer (12), the support (14) including a bar (36), the receiving clip (16) being mounted on the bar (36) of the support (14) by the connecting assembly (18), the receiving clip (16) being slidably mounted along the bar (36) of the support (14);
- positioning the introducer (12) in the central passage (60) through the longitudinal opening (62),
- reversibly moving the locking pad (88) from a released configuration upon placement of the introducer (12) in the central passage (60) to a configuration locking the introducer (12) in position in the central passage (60).
